# EUROPEAN PATENT SPECIFICATION

(11) **EP 1 247 490 B1**
(45) Date of publication and mention of the grant of the patent: **23.07.2008**
(21) Application number: 01900686.5
(22) Date of filing: 12.01.2001
(51) Int. Cl.: A61B 8/00, G01S 7/52, G01S 15/89

(54) **ULTRASONIC DIAGNOSING APPARATUS**
ULTRASCHALLDIAGNOSEGERÄT
APPAREIL DE DIAGNOSTIC ULTRASONORE

(30) Priority: 12.01.2000 JP 2000003727
(43) Date of publication of application: 09.10.2002
(73) Proprietor: HITACHI MEDICAL CORPORATION, Tokyo 100-0047 (JP)
(72) Inventor: SHINOMURA, Ryuichi, Higashimatsuyama-shi, Saitama 355-0004 (JP); MIWA, Yuichi; c/o Central Research Laboratory, Kokubunji-shi, Tokyo 185-8601 (JP); BABA, Hirotaka, Kashiwa-shi, Chiba 277-0005 (JP)
(74) Representative: Strehl Schübel-Hopf & Partner
(86) International application number: PCT/JP2001/000111
(87) International publication number: WO 2001/050961

(56) References cited:
- JP-A- 8 317 923
- JP-A- 11 235 341
- US-A- 4 219 846
- US-A- 4 471 785
- US-A- 4 641 660
- US-A- 5 165 414
- LEVIN NOCK ET AL.: 'Phase aberration correction in medical ultrasound using speckle brightness as a quality factor' THE JOURNAL OF THE ACOUSTICAL SOCIETY OF AMERICAL vol. 85, no. 5, 1989, pages 1819 - 1833, XP002936566

## Description

### FIELD OF THE INVENTION

The present invention relates to an ultrasonic diagnostic apparatus for acquiring an ultrasonic image of a diagnostic part by scanning the interior of an object to be examined in real time with an ultrasonic beam formed with a two-dimensional array of transducers, particularly to an ultrasonic diagnostic apparatus for correcting a focusing error due to a difference of propagating paths of ultrasound transmitted/received with multi-ring type transducers of which a number is grouped into concentric rings.

### BACKGROUND OF THE INVENTION

An apparatus having a plurality of transducers composed of a two-dimensional arrays of transducers and sector scanning an ultrasonic beam (to arbitrary direction) generated with a transmitting/receiving diameter composed with a whole two-dimensional transducer is known. In this ultrasonic diagnostic apparatus, for example if 64 × 64 transducers are arrayed two-dimensionally, then the over all transducer number is 4,096. And an ultrasonic scanning is performed with separate delay control to each transducer element. In this case, a beam-forming circuit with 4,096 channels is necessary. Realizing the beam-forming circuit having these multi-channel delay circuits is difficult. So by thinning out a driven element for forming one ultrasonic beam, the channel number of delay circuits in the beam-forming circuit is reduced. But S/N of a signal acquired by thinning out elements for driving is deteriorated. So said apparatus is realized by comprising a beam-forming circuit having delay channel number as high as possible. For example, there is an apparatus that comprises a beam-forming circuit having a delay circuit of 256 channels for transmitting and 256 channels for receiving.

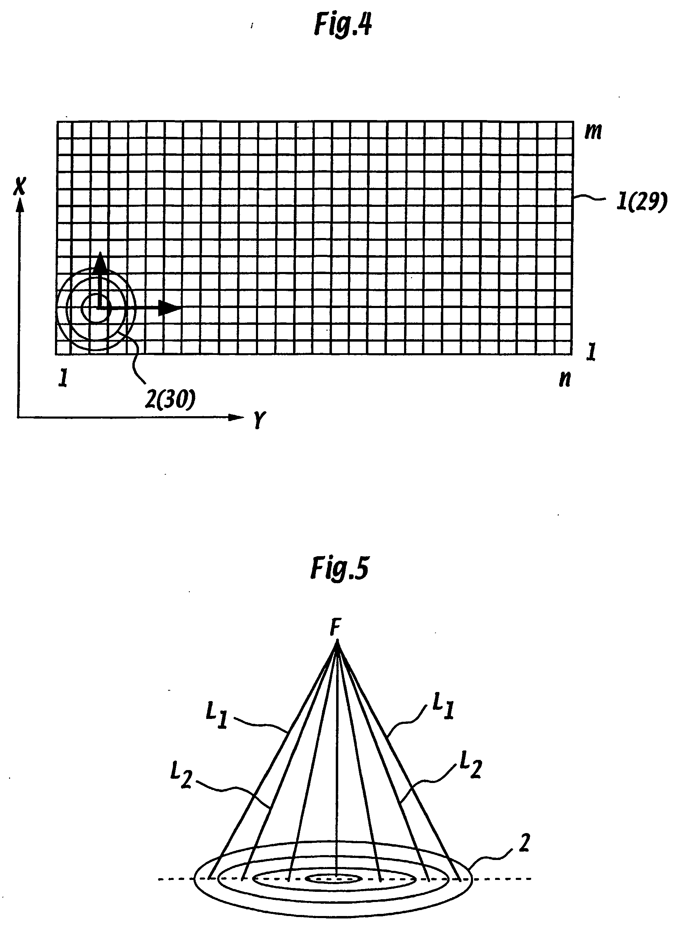

As shown in Fig.4, a linear scanning expanded a field of view by moving a transmitting/receiving diameter in transducer elements 1 arrayed in two dimensions in X, Y direction, and a scanning method applied a convex scanning to two dimensions are proposed. In this case, the number of transducer elements 1 is higher than for said sector scanning type apparatus. So for reducing the channel number in the beam-forming circuit, an apparatus that forms an ultrasound transmitting/receiving diameter 2 by composing a multi-ring having a grouped number of transducer elements 1 in a two-dimensional array with concentric rings, and gives a consistent delay time to transducer elements composing one ring of said multi-ring, and transmits/receives an ultrasonic beam with applying a delay time between rings, and forms an ultrasonic image by moving said diameter 2 to X, Y directions is known.

As shown in Fig.5, in said multi-ring each ring is formed by grouping the transducer elements into concentric rings of which distance from one focal spot F is almost the same L₁, L₂, ···. And the diameter of the most exterior ring is diameter 2 for ultrasound transmitting/receiving. In this method, the grouping of transducer elements in concentric rings is able to reduce greatly the number of channels in the beam forming circuit, which is the number of delay circuits. And S/N of signals acquired by using all elements in a diameter can be improved.

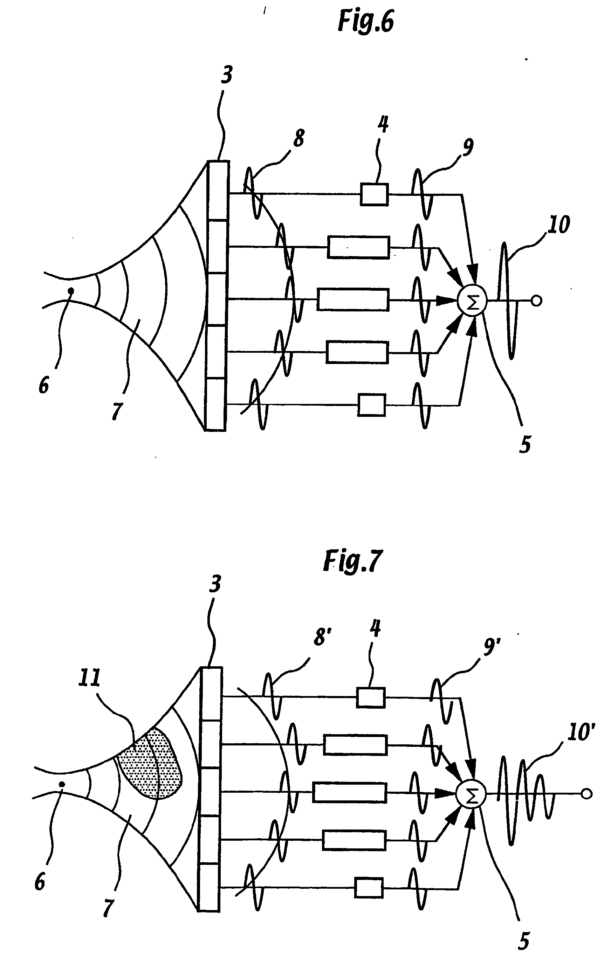

And in case of considering the shape of an ultrasonic beam, focusing calculation of an ultrasonic beam in the object to be examined is traditionally performed under the condition that the sound speed in an ultrasonic propagation medium is uniform. As shown in Fig.6, a traditional apparatus comprises receiving a delay circuit 4, 4, ··· for each transducer element of a probe 3 having a plurality of transducers, and an adder 5 for adding receiving signals output from these receiving delay circuits 4, 4, ···. Although reflected signals from a focus point 6 in the object reach to each transducer element through a propagate medium 7, the difference of the path lengths from the focus point to each transducer element causes differences in the arrival time. In this case, said reflected signals reach early to transducer elements located in the center of the probe 3, and on the other hand reach late to transducer elements in the end of it. So the phase of a wave surface 8 in the receiving signal is not the same. Then the receiving signals output from transducer elements in the center part of probe are delayed with a large amount of delay time in receiving delay circuits 4 corresponding to said each receiving signal, and receiving signals output from transducer elements at the ends of the probe are delayed with a small amount of delay time, and those signals are output to the adder 5. With these delay operations, the wave surface 9 of the receiving signal output from the receiving delay circuits 4 has the same phase. The receiving signal having the same phase such as the wave surface 9 in this condition is added in the adder 5 to form the receiving signal 10.

But actually as shown in Fig.7, a sound speed non-uniformity part 11 exists on course from the focus point 6 in the object to probe 3, so the wave surface of the receiving signal is disturbed as shown in sign 8'. In this case, performing the delay operation assuming the sound speed as to be uniform, the wave surface of receiving signals output from the receiving delay circuit 4 is distorted as shown in sign 9', and does not have the same phase. Accordingly the output added in the adder 5 does not grow in intensity, and its intensity is small such as the receiving signal 10'.

There is a technology referred to as adaptive ultrasonic imaging method to correct a delay amount in said receiving delay circuit 4 in accordance with the sound speed in the medium. As the adaptive ultrasonic imaging technology, a mutual correlation method for correcting a delay amount by correction procession of a received signal between adjacent channels respectively, and a maximum value brightness method for searching a brightness maximum while changing the delay amount of the receiving delay circuits are known.

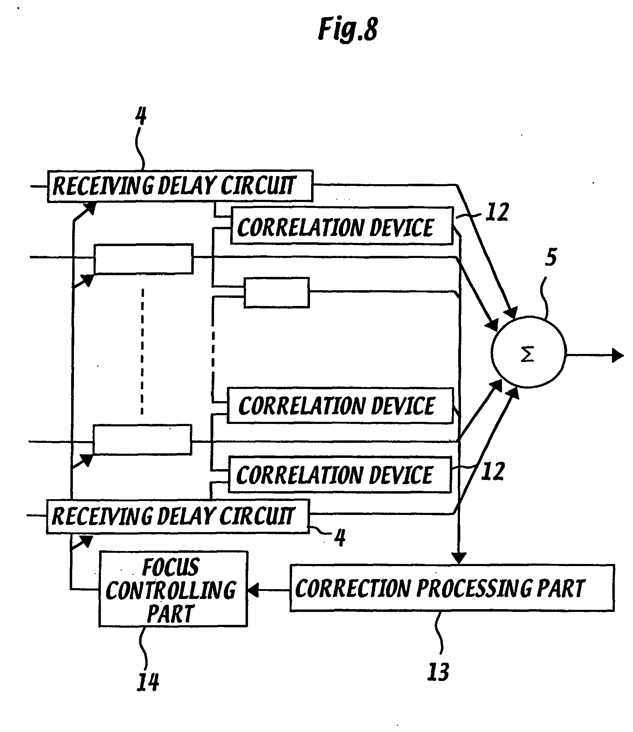

Fig. 8 shows a block diagram describing the mutual correlation method. In Fig.8, a received signal from each transducer element, which is not shown in the figure, is delayed by a predetermined amount with each receiving delay circuit 4, 4,···. This delay is possible by analog delay circuits or a digital delay circuits. In this case, when an output of adjacent channels in each transducer element is performed by mutual correlation processing with correlation device 12, the phase difference between output of adjacent channels can be obtained. Then by detecting the value, and transforming it to focus data in a correction processing part 13, and feed back inputting the transformed data to a focus controlling part 14, a delay amount is corrected with receiving delay circuits 4.

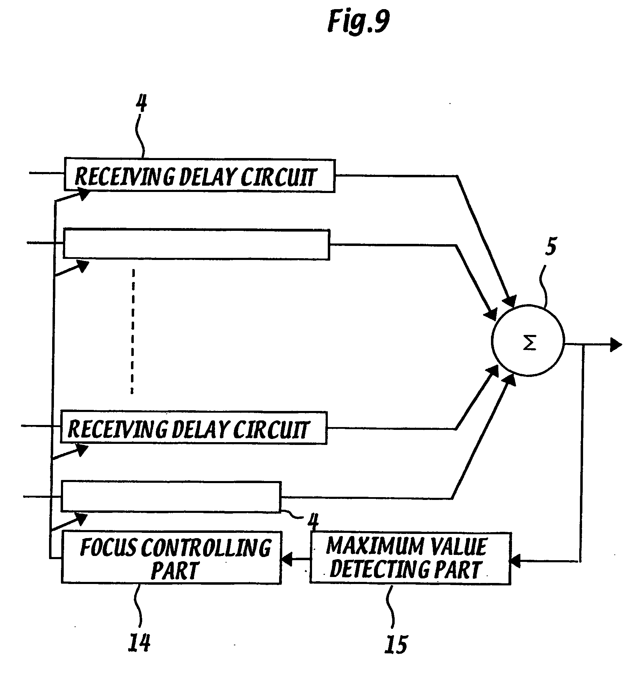

Fig. 9 is a block diagram for describing said maximum value brightness method. In Fig. 9, a received signal from each transducer element, which is not shown in the figure, is delayed in the receiving delay circuits 4, 4, ··· with a predetermined amount. The output delayed in receiving delay circuits 4, 4, ··· is added in the adder 5. And its output is input to a maximum value detecting part 15. This maximum value detecting part 15 compares the input signal with the last input value. And in case the input value is smaller than the last value, focus data is slightly changed systematically in a focus controlling part 14. And the output after phasing of echo signals received with changing this focus data is inputted to the maximum value detecting part 15, and judged again. And after repeating this operation consecutively, when the detected value should converge at a maximum value, its data is used as focus data

However as shown in Fig.4 and Fig.5, in an ultrasonic diagnostic apparatus has a formed transmitting/receiving diameter 2 by grouping two-dimensional array transducer elements 1 in concentric rings to compose a multi-ring. In case that there is a sound speed non-uniformity part 11 on the path from the focus point 6 to the probe 3 in the object, as transducer element 1 in two-dimensional array are grouped in concentric rings as thus described, it was difficult to detect the phase difference due to said path difference for correcting an influence of said sound speed non-uniformity part 11. Therefore, the phase difference of echo signals caused by said path difference with existence of the sound speed non-uniformity part 11 is not corrected. As a result, an image quality is deteriorated because ultrasonic beam becomes worse.

Thus the present invention has to deal with these problems. And the purpose of the present invention is to provide an ultrasonic diagnostic apparatus being able to correct a focusing error by detecting the phase difference of echo signal due to the difference of ultrasonic propagating path, even if its multi-ring is composed by grouping two-dimensional array transducer elements in concentric rings.

Documents US-A-4 471 785 and US-A-4 219 846 disclose ultrasonic imaging systems according to the preamble of claim 1.

### DISCLOSURE OF THE INVENTION

To achieve said object, the ultrasonic diagnostic apparatus of appended claim 1 is provided.

The sublaims relate to preferred embodiments of the present invention.

For example, the diameter of a multi-ring grouped in concentric rings of a two-dimensional array of transducer elements in said probe is divided into a plurality of sections in radial form from the center to the outer. And a focusing error due to sound speed non-uniformity in the object is measured between each divided section of this diameter, and the ultrasonic beam is corrected by feeding back this measured, value to the delay circuits.

Furthermore, according to an embodiment of the present invention, a diameter that is different from that of said multi-ring composed by grouping said transducer element of two dimensional array in said probe in concentric rings is formed. And a focusing error due to a sound speed non-uniformity in the object between each diameter is measured, and the ultrasonic beam is corrected by feeding back this measured value to the delay circuits and returning the form of the multi-ring to its initial setting.

### BRIEF DESCRIPTION OF THE DRAWINGS

Fig.1 is a block diagram of the whole composition showing an embodiment of an ultrasonic diagnosing apparatus according to the present invention.
Fig.2 is an explanation view of a probe showing an illustrative example of the present invention.
Fig.3 is an explanation view of a probe showing an embodiment of the present invention.
Fig.4 is a plan view showing the way a multi-ring is set to the probe, and transmitting and receiving of ultrasonic beam and two-dimensional scanning are performed.
Fig.5 is an explanation view showing a principal of said multi-ring.
Fig.6 is an explanation view showing a traditional formation of an ultrasonic beam.
Fig.7 is an explanation view showing the beam formation in case that a sound speed non-uniformity part is in the medium when said ultrasonic beam is formed.
Fig.8 is a block diagram showing a composition for correcting the delay amount by using a mutual correlation method for an adaptive imaging technology
Fig.9 is a block diagram showing a composition for correcting the delay amount by using a maximum value brightness method for an adaptive imaging technology.

### BEST MODE FOR CARRYING OUT THE INVENTION

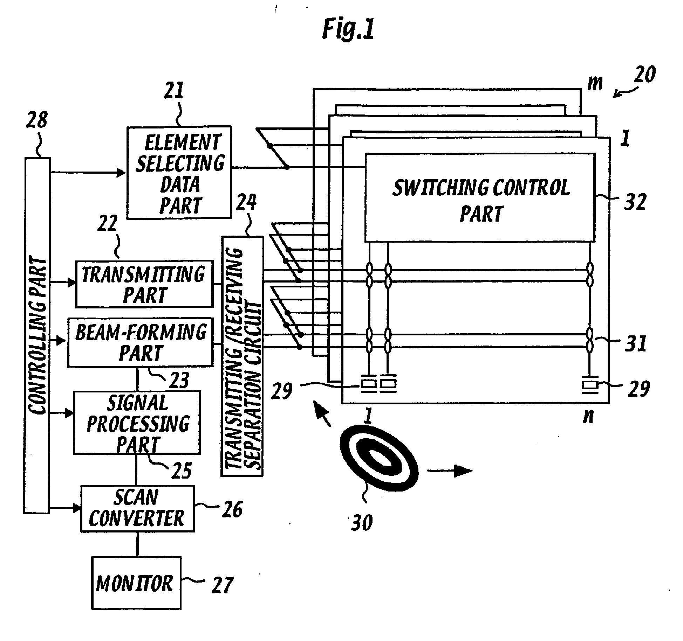

Hereinafter, an embodiment of the present invention and an illustrative example which is not part of the present invention will be explained in detail based on attached drawings FIG.1 is a block diagram of the whole composition showing an embodiment of an ultrasonic diagnostic apparatus according to the present invention. This ultrasonic diagnostic apparatus forms an ultrasonic beam with a two-dimensional transducer array, and acquires an ultrasonic image of a diagnostic part in the interior of an object to be examined by scanning an ultrasonic beam in real time. As shown in Fig.1, it comprises probe 20, an element selecting data part 21, a transmitting part 22, a beam-forming part 23, a transmitting/receiving separation circuit 24, a signal processing part 25, a scan converter 26, a monitor 27, and a controlling part 28.

Said probe 20 transmits ultrasound to an object to be examined and receives its echo. It is composed of a plurality of transducer elements 29, 29, ··· . These transducer elements 29, 29, ··· are arrayed two-dimensionally such as 1~m in x direction and 1~n in y direction in planar view as shown in Fig.4. And said transducer elements are grouped to compose a multi-ring having concentric rings. The multi-ring forms ultrasonic transmitting/receiving diameter 30. A delay time is given between each ring of said multi-ring to transmit and receive the ultrasonic beam. The ultrasonic beam scanning is performed with moving said diameter 30 at each cycle of transmitting/receiving of ultrasound. And an ultrasonic image is formed. In addition, said multi-ring is made for example with fresnel-bundles.

In addition, to said transducers 20, 29. ···, connection switch groups 31 are connected for coupling an arbitrary delay channel in beam-forming circuits described later on and an transducer element. Furthermore, to this connection switch groups 31, a switching control part 32 for controlling the switch operation is connected.

Elements selecting data part 21 memorizes elements selecting data to form the transmitting/receiving diameter 30. And elements selecting data read out from the elements selecting data part 21 is transferred to the switching control part 32. And by controlling said switching control part 32, switch on and off of the connection switch groups 31 is controlled to form the transmitting/receiving diameter 30.

Transmitting part 22 supplies a transmitting signal for an ultrasound to an ultrasonic transducer element respectively with the application of a delay time such that the ultrasound transmitted from each transducer element which forms a transmitting/receiving diameter 30 of said probe 20 is focused to a desired focus point set in the object. And the beam-forming part 23 performs a desired focusing procession respectively to a reflected echo signal received with the transducer elements 29 of said probe 20 and forms a receiving beam by phasing and adding. And the transmitting/receiving separation circuit 24 changes a connection of said transmitting part 22 and the beam-forming part 23 to transducer elements 29, depending on whether an ultrasound is transmitted or received.

Signal processing part 25 inputs the receiving signal from said beam-forming part 23 and obtains a data of one scanning line by processing such as detecting, compression, filtering processing, edge emphasizing etc. And scan converter 26 inputs the data from said signal processing part 25 and forms an image data for displaying to the monitor 27, together performs scan conversion between ultrasonic scanning and scanning for display and interpolation processing of image data or the like. Furthermore, the monitor 27 displays the data from said scan converter 26 as an ultrasonic image. And in the display screen, a three-dimensional image or an arbitrary slice image is displayed. And the controlling part 28 controls an operation of said each composed element

In the present invention, means for measuring a delay error in the receiving signal due to a sound speed non-uniformity part in said the object, and correcting said grouping of the transducer elements in the multi-ring is suggested.

In an illustrative example, the diameter formed in the multi-ring by grouping two-dimensional array transducer element 29, 29, ··· of said probe 20 in concentric rings is divided into a plurality of regions in radial form from the center to the outside. And between each said divided region, the delay error (focusing error) due to the sound speed non-uniformity part (refer to 11 in Fig.7) in the object is measured to correct the beam.

The probe showing this example has transducer elements arranged two-dimensionally. And this is omitted in the figure, but actually they are arrayed in a matrix in the direction of X and Y as shown in Fig.4. Said transducer elements are grouped into ring shape to form diameters of an annular array (multi-ring). Its diameter is moved to X direction or Y direction as a cycle of transmitting/receiving for performing a linear scanning or a convex scanning ultrasonic beam, or a sector scanning beam without moving diameter. With these linear scanning, convex scanning, or sector scanning due to this two-dimensional probe, the image data of three-dimensional volume is acquired from the object. This image data is taken into the scan converter 26 and transformed to the image data. And it is displayed on the screen of the monitor 27 as an ultrasonic slice image or a three-dimensional ultrasonic image.

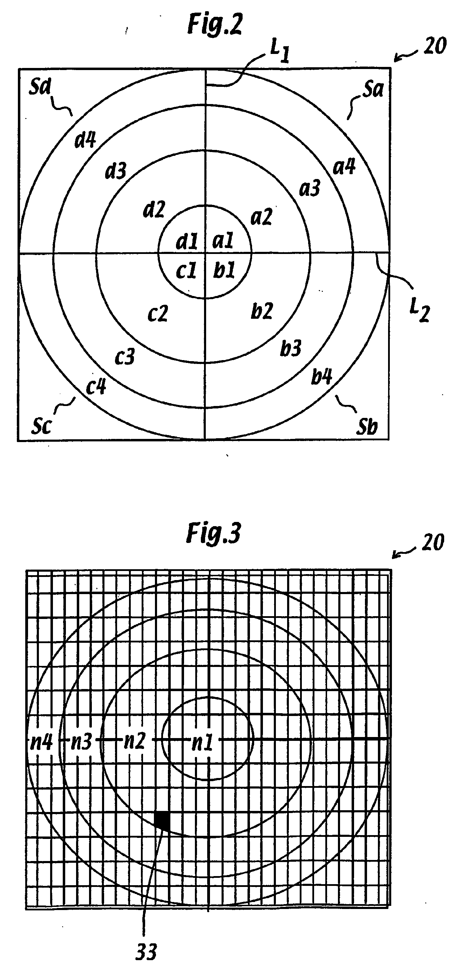

To make it simple, a basic diameter for acquiring one image is shown in Fig.2. In this Fig. 2, the diameter composed of a multi-ring by grouping said two-dimensional array transducer elements in concentric rings is divided into a plurality of regions in radial form from the center to the outer side. For example, elements selecting data is transferred from the elements selecting data part 21shown in Fig.1 to the switching control part 32. With the controlling of said switching control part 32, the dividing line L₁, L₂ of crossing the center of said multi-ring are formed. And this divided line L₁, L₂ divide the multi-ring into four sectors Sa, Sb, Sc, Sd.

Said divided each sector Sa ~ Sd is respectively connected to the transmitting part 22 and the beam-forming part 23 as shown in Fig.1. And to each sector, the delay data calculated in the condition that sound speed is uniform in the medium 7 (refer to Fig.6), is added. That is to say, in Fig.2, the transmitting part 22 and the beam-forming part 23 are connected respectively to ring a₁, a₂, a₃, a₄; b₁, b₂, b₃, b_{4;} c₁, c₂, c₃, c₄; d₁, d₂, d₃, d₄ divided into each sector Sa ~ Sd. And delay controlling is performed respectively. Accordingly, the ultrasonic beam is focused at some point with said delay.

At this time, if the sound speed in the medium 7 is consistent with the sound speed when the delay data is calculated, then the received signal grows with the same phase adding because the phases of the surface in the received signal in after receiving phasing are the same as shown in Fig.6. In addition, if a sound speed non-uniformity part 11 exists in medium 7, then the received signal is small not to grow because the phases of surface in the received signal in after receiving phasing are different from each other as shown in Fig.7.

As shown in Fig.2, the multi-ring is divided into for example four sectors Sa~Sd, so the phase error (this corresponds to focusing error) is calculated with the correlation of received signal after receiving phasing between the channel of transducer elements in each divided sector and corrected as shown in Fig.8. That is to say, to the received signal after said receiving phasing, the phase difference is calculated by mutual correlation between adjacent sectors in each ring divided into each sector Sa~Sd as shown in Fig.2. And the correlation processing is performed between each ring in each sector.

For example, in Fig. 2, in multi-ring divided into four sectors Sa~Sd, mutual correlation processing is performed between adjacent sectors such as a₁ and b₁, b₁ and c₁, c₁ and d₁, d₁ and a₁ in the most interior ring. In addition, the mutual correlation processing is performed between adjacent sectors such as a₂ and b₂, b₂ and C₂, C₂ and d₂ d₂ and a₂ in the second ring. Furthermore also in the third and the fourth ring, the mutual correlation processing is performed between adjacent sectors. And to acquire the relationship between each ring in each sector, the correlation processing between adjacent rings a₁ and a₂, a₂ and a₃, a₃ and a₄ in sector Sa is performed. And also in another sector Sb, the correlation processing is performed between adjacent rings b₁ and b₂, b₂ and b₃, b₃ and b₄. Furthermore, also in other sector Sc, Sd, the correlation processing is performed between adjacent rings. In addition, a method of correlation processing is not restricted to the above described. Other methods can be used.

On the other hand, it is preferable to correct the phase error by calculating the delay amount that brings maximum or lightness with changing delay amounts of the beam-forming part between channels of transducer elements in each divided sector in the same way as shown in Fig.9, in the condition that for example multi-ring is divided into four sectors Sa~Sd as shown in Fig.2. That is, it is preferable to correct the phase error by calculating the delay amount that brings maximum or brightness with changing delay amounts consecutively to the ring, which is divided into each sector Sa~Sd, a₁, a₂, a₃, a₄; b₁, b₂, b₃, b₄; c₁, c₂, c₃, c₄; d₁, d₂, d₃, d₄.

Next an embodiment of the present invention will be described. In this embodiment, it is preferable to form a diameter being different from the diameter composed by grouping transducer elements arrayed two-dimensionally in said probe 20 to multi-ring in concentric rings, and measure the delay error due to a sound speed non-uniformity part in the object between each diameter (refer to 11 in Fig.7), and correct the beam.

Fig.3 is an explanation view of a probe showing the embodiment. This probe has a two-dimensional array of transducer elements. Each transducer element is connected to a receiving delay circuit. An ideal delay is given for some focus point to calculate the delay error by correlating the outputs of the transducer elements. In the same way as shown in Fig.5, when a multi-ring is grouped, if the grouped transducer elements forming one ring for example have a difference of the reaching time (distance) of ultrasound to a focus point F in the range of λ/10 (λ is wave-length of ultrasonic beam), then the delay error is corrected considering said calculated delay error to the reaching time.

For example, it is assumed that a specified transducer element 33 has a delay error in multi-ring n1, n2, n3 , n4 shown in Fig.3. The transducer element 33 is grouped into ring n2 in ideal state, but when the delay error is considered, the correction is performed such that it is grouped into ring n3. The delay error is corrected, and after having calculated a correction value of the delay error, the transducer element 33 is grouped to return the ring into the original ideal state. Thus, not the diameter for forming an ultrasonic beam actually (grouped ring n1~n4), but the delay error is detected with transducer elements in another grouped ring. And the correction is performed to make a grouped ring considering the delay error.

In case of the above, if a transmitter and a beam-forming circuit are connected to all of the two-dimensional array transducer elements as shown in Fig.3, then the circuit scale is large. So for example it is preferable to group adjacent transducer elements into rectangular blocks, and connect the transmitter and the beam-forming circuits to grouped transducer elements as a unit. In addition, also in the embodiment, it is preferable to correct the phase error by calculating the delay amount in which the output signal is maximum with a changing delay amount of the beam-forming part as shown in Fig.9.

As thus described, the present invention comprises means for measuring a focusing error due to a sound speed non-uniformity part in the object and correcting the grouping and delay time of a multi-ring having a two-dimensional array of transducer elements in concentric rings. And the interior of the object is imaged with the corrected beam, so that the image quality of the ultrasonic image is improved.

## Claims

1. An ultrasonic diagnostic apparatus comprising:
transducer selection means (21, 32) for assigning transducer elements (29) of a two-dimensional array to a plurality of rings;
a beam-forming part (23) comprising delay circuits (4) for applying a delay time to each of said rings and an adder circuit (5) for adding an output of each of said delay circuits (4);
means (27) for imaging the output signal of said beam-forming part (23),
**characterized by**:
means for detecting a transducer element having a focusing error in each of said rings; and
ring correction means adapted for assigning said transducer element which has been detected to have a focusing error to a ring different from the ring it has initially been assigned to.

2. The apparatus of claim 1, further comprising:
a probe (20) including said two-dimensional array transducer elements (29) for transmitting and receiving an ultrasound signal to an object to be examined, wherein said ultrasound beam is applied with a delay time between each of said rings;
means (24) for transmitting and receiving ultrasonic beams corrected by said ring correction means based on a measured focusing error due to a sound speed non-uniformity interior of said object and modifying the assigning of said transducer elements (29) or the delay time based on the measured error; and
means for imaging the object with an echo signal of the corrected ultrasonic beam.

3. The apparatus of any preceding claim, further comprising;
means for dividing said plurality of rings into a plurality of sections,
means (13) for calculating a focusing error between said sections, and
means (14) for feed-back correcting said calculated focusing error to the delay time set in said delay circuit (4).

4. The apparatus of claim 3, wherein
said sections have radial shape from the center outwards, and
said focusing error is due to a sound speed non-uniformity in different parts of the object.

5. The apparatus of claim 3 or 4, wherein said means (13) for calculating the focusing error between said sections includes means for calculating the focusing error between rings in one section.

## Patentansprüche

1. Ultraschalldiagnosegerät mit
einer Wandlerauswähleinrichtung (21, 32) zum Zuordnen von Wandlerelementen (29) eines zweidimensionalen Arrays zu mehreren Ringen,
einem Strahlformungsabschnitt (23) mit Verzögerungsschaltungen (4) zum Anlegen einer Verzögerungszeit an jeden der Ringe und mit einer Addierschaltung (5) zum Addieren eines Ausgabesignals von jeder der Verzögerungsschaltungen (4),
einer Einrichtung (27) zum Abbilden des Ausgabesignals des Strahlformungsabschnitts (23),
**gekennzeichnet durch**
eine Einrichtung zum Erfassen eines Wandlerelements mit einem Fokussierfehler in jedem der Ringe, und
eine Ringkorrektureinrichtung, die dazu ausgelegt ist, das Wandlerelement, das als mit einem Fokussierfehler behaftet erfasst wurde, einem anderen Ring zuzuordnen als dem, dem es ursprünglich zugeordnet worden war.

2. Gerät nach Anspruch 1, ferner mit
einer Sonde (20), die den zweidimensionalen Array von Wandlerelementen (29) aufweist, zum Senden und Empfangen eines Ultraschallsignals an ein zu untersuchendes Objekt, wobei der Ultraschallstrahl mit einer Verzögerungszeit zwischen jedem der Ringe zugeführt wird,
einer Einrichtung (24) zum Senden und Empfangen von Ultraschallstrahlen, die durch die Ringkorrektureinrichtung auf Grundlage eines gemessenen Fokussierfehlers korrigiert wurden, der aus einer Schallgeschwindigkeitsungleichmäßigkeit im Inneren des Objekts resultiert, und zum Modifizieren der Zuordnung der Wandlerelemente (29) oder der Verzögerungszeit auf Grundlage des gemessenen Fehlers, und
einer Einrichtung zum Abbilden des Objekts mit einem Echosignal des korrigierten Ultraschallstrahls.

3. Gerät nach einem der vorstehenden Ansprüche, ferner mit
einer Einrichtung zum Unterteilen der mehreren Ringe in mehrere Bereiche,
einer Einrichtung (13) zum Berechnen eines Fokussierfehlers zwischen den Bereichen, und
einer Einrichtung (14) zur Feedback-Korrektur des berechneten Fokussierfehlers bezüglich der in der Verzögerungsschaltung (4) eingestellten Verzögerungszeit.

4. Gerät nach Anspruch 3, wobei
die Bereiche radiale Form von der Mitte nach außen aufweisen, und
der Fokussierfehler aus einer Schallgeschwindigkeitsungleichmäßigkeit in verschiedenen Teilen des Objekts resultiert.

5. Gerät nach Anspruch 3 oder 4, wobei die Einrichtung (13) zum Berechnen des Fokussierfehlers zwischen den Bereichen eine Einrichtung zum Berechnen des Fokussierfehlers zwischen Ringen in einem Bereich aufweist.

## Revendications

1. Dispositif de diagnostic par ultrasons comportant :
des moyens de sélection de transducteurs (21, 32) pour attribuer des éléments transducteurs (29) d'un réseau bidimensionnel à une pluralité d'anneaux,
une partie de formation de faisceau (23) comportant des circuits à retard (4) pour appliquer un temps de retard à chacun desdits anneaux et un circuit additionneur (5) pour additionner une sortie de chacun desdits circuits à retard (4),
des moyens (27) pour reproduire le signal de sortie de ladite partie de formation de faisceau (23),
**caractérisé par** :
des moyens pour détecter un élément transducteur ayant une erreur de focalisation dans chacun desdits anneaux, et
des moyens de correction d'anneau adaptés pour attribuer ledit élément transducteur qui a été détecté comme ayant une erreur de focalisation, à un anneau différent de l'anneau auquel il a été initialement attribué.

2. Dispositif selon la revendication 1, comportant en outre :
une sonde (20) incluant lesdits éléments transducteurs de réseau bidimensionnel (29) pour transmettre un signal à ultrasons à un objet à examiner et recevoir un signal à ultrasons en provenance de celui-ci, ledit faisceau d'ultrasons étant appliqué avec un temps de retard entre chacun desdits anneaux,
des moyens (24) pour transmettre et recevoir des faisceaux d'ultrasons corrigés par lesdits moyens de correction d'anneau sur la base d'une erreur de focalisation mesurée due à un intérieur dudit objet ayant une non-uniformité de termes de vitesse du son, et pour modifier l'attribution desdits éléments transducteurs (29) ou le temps de retard sur la base de l'erreur mesurée, et
des moyens pour reproduire l'objet à l'aide d'un signal d'écho du faisceau d'ultrasons corrigé.

3. Dispositif selon l'une quelconque des revendications précédentes, comportant en outre :
des moyens pour diviser ladite pluralité d'anneaux en une pluralité de tronçons,
des moyens (13) pour calculer une erreur de focalisation entre lesdits tronçons, et
des moyens (14) pour corriger par rétroaction ladite erreur de focalisation calculée dans le temps de retard défini dans ledit circuit à retard (4).

4. Dispositif selon la revendication 3, dans lequel
lesdits tronçons ont une forme radiale depuis le centre vers l'extérieur, et
ladite erreur de focalisation est due à une non-uniformité de la vitesse du son dans différentes parties de l'objet.

5. Dispositif selon la revendication 3 ou 4, dans lequel lesdits moyens (13) pour calculer l'erreur de focalisation entre lesdits tronçons incluent des moyens pour calculer l'erreur de focalisation entre des anneaux dans un tronçon.
